# EUROPÄISCHE PATENTSCHRIFT

(11) **EP 3 544 654 B1**
(45) Veröffentlichungstag und Bekanntmachung des Hinweises auf die Patenterteilung: **03.01.2024**
(21) Anmeldenummer: 17825917.2
(22) Anmeldetag: 27.11.2017
(51) Int. Cl.: A61M 3/02, A61F 2/00, A61F 5/442

(54) **VORRICHTUNG FÜR DIE REPETITIVE ZU- UND ABLEITUNG VON SUBSTANZEN FÜR DIE MEDIZINISCHE THERAPIE**
DEVICE FOR THE REPETITIVE SUPPLY AND DRAINING OF SUBSTANCES FOR MEDICAL THERAPY
DISPOSITIF POUR L'INTRODUCTION ET L'ÉVACUATION RÉPÉTITIVE DE SUBSTANCES POUR LA THÉRAPIE MÉDICALE

(30) Priorität: 25.11.2016 DE 102016014053
(43) Veröffentlichungstag der Anmeldung: 02.10.2019
(73) Patentinhaber: Advanced Medical Balloons GmbH, 68753 Waghäusel (DE)
(72) Erfinder: GÖBEL, Fred, 67346 Speyer (DE)
(74) Vertreter: Küchler, Stefan
(86) Internationale Anmeldenummer: PCT/IB2017/001458
(87) Internationale Veröffentlichungsnummer: WO 2018/096392

(56) Entgegenhaltungen:
- EP-A2- 0 254 410
- WO-A1-2009/144028
- DE-A1-102012 003 034
- DE-C- 338 976

## Beschreibung

Die Erfindung richtet sich auf eine Vorrichtung zur Durchführung einer lavage-artigen, repetitiven Zu- und Ableitung einer spülenden, reinigenden oder sonstigen therapeutisch wirksamen Substanz zu/von einem Patienten, insbesondere im Rahmen einer irrigierenden Entleerung des Colon eines Patienten, wobei die Vorrichtung eine dauerhaft atraumatisch anorektal platzierbare, stuhlaufnehmende und -ableitende Kathetereinheit aufweist, ferner eine Beutel- bzw. Beutel-/Schlaucheinheit mit kombinierter Spül- und Sammelfunktion, sowie eine die Kathetereinheit mit der Spül- und Sammelbeuteleinheit verbindende Schlaucheinheit, umfassend einen ein- oder mehrlumigen, folienartigen, flach kollabierbaren Schlauch.

Die Spülung (Irrigation) des Dickdarms (Colon) bzw. des Enddarms (Rektum) eines Patienten mittels kombiniert zu- und ableitender Kathetersysteme ist gängiger Bestandteil der medizinischen Praxis und wird bei verschiedensten Indikationen angewandt.

Die bisher bekannte Bauart von Spülsystemen für die colo-rektale Irrigation ist jedoch in mehrfacher Hinsicht unbefriedigend. Insbesondere muss bei konventionellen Irrigationssystemen der trans-anal, im Rektum platzierte Katheteranteil im unmittelbaren Anschluss an die colo-rektale Spülung entfernt werden. Bedingt durch die in der Regel rigide, die komfortable trans-anale Einführung unterstützende Bauart des Katheterschaftes, kann es im Falle darmwärts gerichteter Dislokationen des Katheters zu einer traumatischen Perforation der Darmwandung durch den Schaft kommen. Die nicht gewährleistete Option einer längerfristigen, Traumata ausschließenden Positionierung des Katheters im Rektum ist im klinischen Einsatz insofern problematisch, als Darminhalt durch die Irrigation zwar mobilisiert wird, sich im Anschluss an die Irrigation aber häufig nur verzögert in vielen nachfolgenden Fraktionen entleert. Die zum Teil über viele Stunden anhaltenden Nachfolgestühle gehen in der Regel mit einer erheblichen Verschmutzung des Patienten einher.

Zur Reinigung bzw. Entleerung des Darms bzw. zur Mobilisierung von festem Stuhl aus dem Rektum bzw. Colon eines Patienten werden in der Regel sogenannte Schwenkeinläufe durchgeführt. Bei diesem Verfahren wird Spülflüssigkeit in einen Behälter gefüllt, der über ein Schlauchsystem mit dem trans-anal eingeführten, in das Rektum reichenden und dort platzierten, in der Regel mit einem Halteballon gesicherten Spülkatheter verbunden ist. Bei der Zuführung des Mediums wird der Behälter über das Patientenniveau angehoben und zur der Ableitung des mobilisierten Darminhaltes unter Patientenniveau abgesenkt. Das Verfahren wird so lange wiederholt, bis sich der aus dem Darm auszuwaschende Stuhl soweit im Spülmedium gelöst bzw. suspendiert hat, dass er bei der abschließenden Entleerung von Darminhalt in den aufnehmenden Behälter größtmöglich abgeleitet werden kann. Für eine effiziente Suspension von Stuhl sind mehrere aufeinanderfolgende Hebe- und Senkzyklen erforderlich, wodurch sich das Verfahren in der Regel langwierig gestaltet. Bei der mehrfachen Wiederholung des Zyklus kommt es zudem vermehrt zum Eintrag von Spülflüssigkeit in höhere Colon-Abschnitte, was in zu einer weiteren Verzögerung der Ausscheidung des mobilisierten Darminhaltes führt.

Aus der WO 2009/144028 A1 ist eine Vorrichtung zum Abdichten einer natürlichen oder künstlichen Öffnung des Dickdarms (Colon) bzw. Enddarms (Rektum) eines Patienten sowie zum Verschließen derselben und/oder zur Entfernung von Stuhl daraus, insbesondere zur kontinuierlichen Drainage und/oder zur intermittierenden Irrigation, vorzugsweise in ein externes, beutelartiges Auffanggefäß, bekannt, umfassend einen aufblasbaren Ballon mit einer etwa ringförmigen Struktur, gebildet aus einem flächigen, in sich umgestülpten Schlauchabschnitt, wobei die äußere Lage des umgestülpten Schlauchabschnittes einen radial erweiterten Bereich zum Einführen in das Rektum und einen demgegenüber verjüngten Bereich aufweist, der während des Gebrauchs zumindest bereichsweise außerhalb des Rektums verbleibt, wobei der intrarektale Ballonabschnitt in keiner funktionellen oder räumlichen Verbindung mit dem transanalen Ballonabschnitt steht. Eine Anordnung mit einer kombinierten Spül- und Sammelbeuteleinheit ist dort allerdings nur in Verbindung mit einem Konnektorelement in Form eines konischen Steck-Anschlusses offenbart, der aufgrund seiner massiven Wandstärke nicht biegsam ausgebildet ist und demzufolge seine Geometrie in Abhängigkeit von der Durchflussrichtung und/oder -geschwindigkeit nicht ändert. Demzufolge erfolgt die Zu- und Ableitung von Flüssigkeit zu/von dem Patienten gleichermaßen, also mit gleichen oder vergleichbaren Strömungsmengen und - geschwindigkeiten. Aufgrund des großen Querschnitts der Schlaucheinheit zwischen der Spül- und Sammelbeuteleinheit einerseits und der Kathetereinheit andererseits erfolgt die Zuleitung daher schwallartig und somit auf irritierende bzw. nicht organverträgliche Weise.

Es wäre daher ein System wünschenswert, welches die Anzahl der erforderlichen Spül- und Ableitungszyklen möglichst minimiert. Das System sollte dabei insgesamt für eine rasche, effiziente Auswaschung von Darminhalt, insbesondere auch von geformten Stuhlportionen geeignet sein, während jedoch die Zuleitung der Drainageflüssigkeit in einer organverträglichen Weise erfolgen sollte. Ferner sollte das Irrigationssystem nach Beendigung des Spülvorganges über mehrere Stunden bis eventuell auch Tage im Patientenrektum verbleiben können, um durch die Irrigation mobilisierten, verzögert ausgeschiedenen Darminhalt aufnehmen bzw. in ein geschlossenes Auffangsystem leiten zu können.

Die Erfindung wird durch die Merkmale des unabhängigen Anspruchs 1 definiert.

Die Lösung dieses Problems gelingt im Rahmen einer gattungsgemäßen Vorrichtung, umfassend eine dauerhaft atraumatisch anorektal platzierbare, stuhlaufnehmende und -ableitende, vorzugsweise den Analkanal über seine gesamte Länge hinweg durchsetzende und abdichtende Kathetereinheit, eine Spül- und Sammelbeuteleinheit mit kombinierter Spül- und Sammelfunktion, sowie eine die Kathetereinheit mit der Spül- und Sammelbeuteleinheit verbindende Schlaucheinheit, umfassend einen ein- oder mehrlumigen, folienartigen, flach kollabierbaren Schlauch, durch wenigstens ein Element mit einer flussrichtungsabhängigen Drosselfunktion, wobei die Ableitung von Darminhalt vom Patienten her derart großlumig erfolgt, dass auch geformte Stuhlanteile effizient abgeleitet werden, während die Zuleitung von Flüssigkeit zum Patienten hin im Verhältnis zu der großlumigen Ableitung von Darminhalt gedrosselt erfolgt und somit die Strömungsmenge oder -geschwindigkeit bei der Zuleitung auf ein organverträgliches, nicht-irritierendes Maß reduziert.

Im Rahmen der Anmeldung kann/sollte der Begriff "atraumatisch platzierbar" dahingehend interpretiert werden, dass er frei von einem rigiden, schaftartigen, tragenden Körper ist. ferner kann/sollte der Begriff "anorektal platzierbar" bedeuten, dass der betreffende Körper durch den Anus im Rektum platziert wird. Als "flussrichtend" soll eine Funktion verstanden werden, welche je nach Flussrichtung unterschiedliche Strömungen, insbesondere Strömungsmengen, zulässt. Als Drosselung soll eine Reduzierung der Strömungsmenge oder - geschwindigkeit verstanden werden.

Diese erfindungsgemäße Struktur macht es möglich, für die Aufnahme der Spülflüssigkeit und für die Aufnahme des ausgewaschenen Darminhalts den selben Beutel zu verwenden, weil ein Rückstrom bereits ausgewaschener Stuhlanteile in den Patienten ganz oder weitgehend ausgeschlossen ist.

Für die effiziente Ableitung geformter Stuhlanteile sind alle Segmente des Kathetersystems bevorzugt mit einem großen, für die Passage des Stuhls ausreichenden Durchmesser ausgestattet. Im Rahmen der Erfindung werden funktionelle Komponenten beschrieben, die geformte Stuhlanteile aus dem repetitiv zugeführten Spülmedium im System abscheiden, so dass lediglich wässrig suspendierter Stuhl zum Patienten zurückfließt. Die effiziente Ableitung und Abscheidung geformter Stuhlportionen reduziert die Anzahl der Spülungszyklen im Wesentlichen dadurch, dass die Stuhlfragmente nicht im Darm des Patienten in eine wässrige Suspension gebracht werden müssen um drainiert werden zu können, wie dies bei konventionellen Systemen mit in der Regel klein-lumigem Katheterschaft erforderlich ist.

Eine vollständige oder partielle bzw. abscheidende Trennung von zugeführtem Spülmedium und abgeleitetem Darminhalt kann durch entsprechende Funktionselemente sowohl im kopfteiligen, im trans-anal platzierten Segment, im zu- bzw. ableitenden Schlauchsegment zwischen Kopfteil und Behälter, oder auch im Behälter selbst integriert sein.

Großlumig ausgelegte, trans-anal platzierte, colo-rectal spülende Kathetersysteme sind bekannt, jedoch in ihrer Anwendung insbesondere im Bereich des trans-analen Kathetersegmentes problematisch. Deren typischerweise relativ rigide ausgeführten Schaftelemente weiten den analen Schließmuskel und gehen zudem mit dem Risiko einer Perforation der exponierten Darmanteile einher.

Bei groß-lumigen Spül- bzw. Einlaufsystemen kann ein zu rascher, nicht modulierbarer Einstrom von Spülmedium in den Darm ebenfalls problematisch sein. Flutet ein zu großes Volumen zu rasch in den Darm, kann dies schmerzhafte Sensationen bis hin zu spasmenartige Kontraktionen auslösen. Wünschenswert wäre daher eine den Fluss zum Patienten hin begrenzende Funktion, die im einfachsten Falle durch eine drosselnde Bemaßung in den zuleitenden Komponenten ermöglicht werden kann.

Ein weiteres Problem bei der konventionellen colo-rektalen Irrigationen zeigt sich beim Einlauf größerer Spülvolumina.

Große Einlaufmengen können von vielen Patienten, z.B. wegen eines schlechten Allgemeinzustandes oder wegen einem schwachen Schließmuskel nicht sicher im Darm gehalten werden. Zwar kann der Patient durch Kontraktion des Schließmuskels anfangs eine Leckage von Darminhalt verhindern, jedoch ermüdet der Sphinkter in der Regel nach einigen Minuten. Es kommt dann zum Herausrinnen von Darminhalt am trans-anal platzierten Schaft des Spülkatheters vorbei.

In der bevorzugten Ausführungsform der vorliegenden Erfindung weist der Katheter eine Kopfeinheit auf, die für das dauerhafte, atraumatische Verbleiben im Rektum konzipiert ist. Als baulicher Grundtyp kommen hierbei prinzipiell handelsübliche Stuhldrainagen bzw. Fecal Management Systeme für die kontinuierliche Stuhlableitung in Frage. Diese weisen einen einfachen, dem Rektumboden ankernd aufliegenden Retentionsballon von toroidaler Gestalt auf. Dem Ballon schließt sich in Verlängerung ein einfacher, trans-anal stuhlableitender Schlauch an, der sich im Analkanal radial faltet bzw. einstülpt.

Ferner können im Rahmen der Erfindung Kopfeinheiten mit besonderer trans-analer Ballondichtung zum Einsatz kommen, die beispielsweise in der EP 1 784 140 B1, der WO 2013 / 026 564 A1 oder der DE 10 2008 055 674 A1 beschrieben wurden. Diese weisen neben einem hantelförmigen Ballon für die kombinierte rektale Retention und trans-anale Dichtung auch eine den Ballon tragende, axial und radial stauchbare, trans-anal positionierte Schaftkomponente auf, die den Stuhl rektal aufnimmt und durch den Anus hindurchleitet. Die beschriebene Bauform hat sich als besonders atraumatisch erwiesen und gestattet Verweilzeiten von mehreren Stunden bis Tagen. Die Kopfeinheiten können sich zudem an die jeweilige Öffnung des Sphinkters anpassen und eine so eine dynamische wirkende, sehr effiziente Dichtung des Analkanals gewährleisten.

An die Kopfeinheit schließt sich erfindungsgemäß ein extrakorporaler, zu- und ableitender Schlauch an, der endständig mit einem Spül- bzw. Sammelbeutel verbunden ist. Der ableitende Schlauch ist in seiner bevorzugten Ausführung folienartig dünn, so dass zwar im befüllten Zustand ein großes Drainagelumen erreicht werden kann, der Schlauch aber im nicht befüllten Zustand zu einer flachen, bandartigen Struktur kollabiert und den Patienten so optimal wenig irritiert bzw. Druckstellen beim eventuellen Aufliegen des Patienten auf dem Schlauch verhindert.

Das die Kopfeinheit mit dem Spül-und Sammelbeutel verbindende Schlauchsegment kann ein oder auch mehrere Lumen aufweisen. Diese können beispielsweise konzentrisch zueinander angeordnet, oder auch parallel angeordnet sein. Bevorzugt bestehen beide Strukturen aus einer kollabierbarer Schlauchfolienstruktur. Die doppelläufige Anordnung ermöglicht es die zugeleitete Flüssigkeit vom abgeleiteten Darminhalt vollständig zu trennen. In diesem besonderen Falle münden beide Lumina am distalen Ende der Vorrichtung etwa auf gleicher Höhe. Werden flussrichtende Komponenten in die jeweils zu- und ableitenden Schlauchanteile verbaut, kann die zugeleitete Flüssigkeit nahezu vollständig vor einer Kontamination mit bereits drainiertem Darminhalt oder mit von Darminhalt kontaminierten Flächen geschützt werden.

Eine wesentliche Baugruppe des in der Erfindung vorgestellten Spülsystems stellt der Behälter dar, der die Einlaufflüssigkeit bevorratet bzw. den vom Patienten ausgeschiedenen Darminhalt aufnimmt. Die Erfindung schlägt hier sowohl ein ein-kammerige, als auch ein zwei-kammerige Systeme vor.

In der Ausführung mit einer Kammer ist bevorzugt ein stuhlabscheidendes System vorgesehen, das geformte Stuhlanteile zurückhält und nur den Einstrom suspendierter Stuhlpartikel in den Darm erlaubt. Der Behälter wirkt sozusagen als Stuhlabscheider. In der zwei- kammerigen Ausführung enthält ein Kompartiment des Behälters die Spülflüssigkeit, während das andere Kompartiment, vollständig vom ersten separiert, den drainierten Darminhalt aufnimmt

Zur Richtung der jeweiligen Flüsse sind sowohl das ein- als auch das zwei-kammerige Behältersystem mit flussrichtenden Ventilmechanismen versehen. Eine Flussrichtungsfunktion kann optional in das verbindende Schlauchsystem zwischen Katheterelement und Behälter integriert werden. Auch ein Einbau in der trans-anal platzierten Kopfeinheit ist denkbar. Bei den zu verwendenden Komponenten geht die Erfindung von besonders leicht gebauten, atraumatisch kollabierbaren, bevorzugt folienartigen Komponenten mit Flussrichtungsfunktion aus.

Die Erfindung beschreibt eine Vorrichtung und ein Verfahren zur Durchführung einer lavage-artigen, repetitiven Zu- und Ableitung einer spülenden, reinigenden oder sonstigen therapeutisch wirksamen Substanz, insbesondere im Rahmen einer irrigierenden Entleerung des Colon eines Patienten, wobei die Vorrichtung eine Beutel- bzw. Beutel-/Schlaucheinheit mit kombinierter Spül- und Sammelfunktion aufweist, die über mindestens eine flussrichtende Funktion verfügt, die den unmittelbaren Rückstrom von bereits in den Beutel abgeleiteter Spülflüssigkeit zum Patienten hin ausschließt, und darüber hinaus im Beutel die Abscheidung geformter Anteile aus der bereits abgeleiteten Spülflüssigkeit erlaubt. Die Kombination der erfindungsgemäßen Beutel- bzw. Beutel-/Schlaucheinheit mit einer atraumatischen, trans-anal platzierten, den Analkanal vorzugsweise durch eine hantelförmige Ballonkomponente dichtenden Katheter-Einheit, ermöglicht groß-volumige Einläufe, die eine nahezu vollständige Entleerung bzw. Lavage des Colon ermöglichen, ohne dass es während des Einlaufs zur Leckage von Spülflüssigkeit aus dem Anus kommt. Die atraumatische Platzierbarkeit der Kathetereinheit gestattet es ferner, den Katheter nach Abschluss der Lavage im Patientenrektum zu belassen, und erst nach vollständiger Entleerung des Colons zu entfernen, wodurch Verschmutzungen des Patienten durch nachfolgende Stuhlentleerungen vermieden werden können. Bei dauerhafter trans-analer Platzierung der Kathetereinheit kann diese für eine intermittierende Entleerung bzw. Lavage verwendet werden, die das absteigende Colon bzw. das Rektum von Stuhl freihält, und so den Zustand einer Quasi-Kontinenz ermöglicht. Im Intervall zwischen den Spülungen gewährleistet die im Anus platzierte Kathetereinheit eine kontinuierliche Dichtung des Anus. Sie wird dann mit einem besonders leicht gebauten, folienartigen, entgasenden und/oder sekretsammelnden Element abgeschlossen. Die Konnektion der spülenden Beutel-/Schlaucheinheit sowie der abschließenden, entgasenden Einheiten erfolgt durch eine atraumatische, flach kollabierbare Konnektoreinheit, die die Entstehung von Druckstellen vermeidet. Weitere Merkmale, Einzelheiten, Vorteile, Wirkungen und funktionelle Aspekte der Erfindung ergeben sich aus der nachfolgenden Beschreibung mehrerer Ausführungsformen der Erfindung sowie anhand der beiliegenden Zeichnung. Hierbei zeigt:
- Abb.1: eine erfindungsgemäße Vorrichtung mit einem ein-kammerigen, kombinierten Irrigations- und Sammelbehälter mit flussrichtungsgetrennter Zu- und Ableitung, sowie mit einer Retentionseinheit für die Stuhlaufnahme und Verankerung der Vorrichtung im Rektum;
- Abb.2a: ein zwei-lumiges Schlauchelement aus dünnwandiger, leicht kollabierbarer Schlauchfolie, mit parallelem Verlauf der Einzellumina;
- Abb. 2b: ein zwei-lumiges Schlauchelement aus dünnwandiger, leicht kollabierbarer Schlauchfolie, mit konzentrischem Verlauf der Einzellumina;
- Abb.3: eine bevorzugte, einkammerige Ausführungsform der erfindungsgemäßen Vorrichtung für die repetitive Zu- und Ableitung einer Substanz, mit integrierter Abscheidefunktion von geformten, bereits in den Sammelbehälter abgeleiteten Bestandteilen, im Verbund mit einer bevorzugten, trans-anal dichtenden Kathetereinheit;
- Abb. 4: ein vollständig getrenntes, zwei-kammeriges Spül-/Sammelbeutelsystem mit integrierten Flussrichtungsfunktionen im Auslassbereich des Beutels;

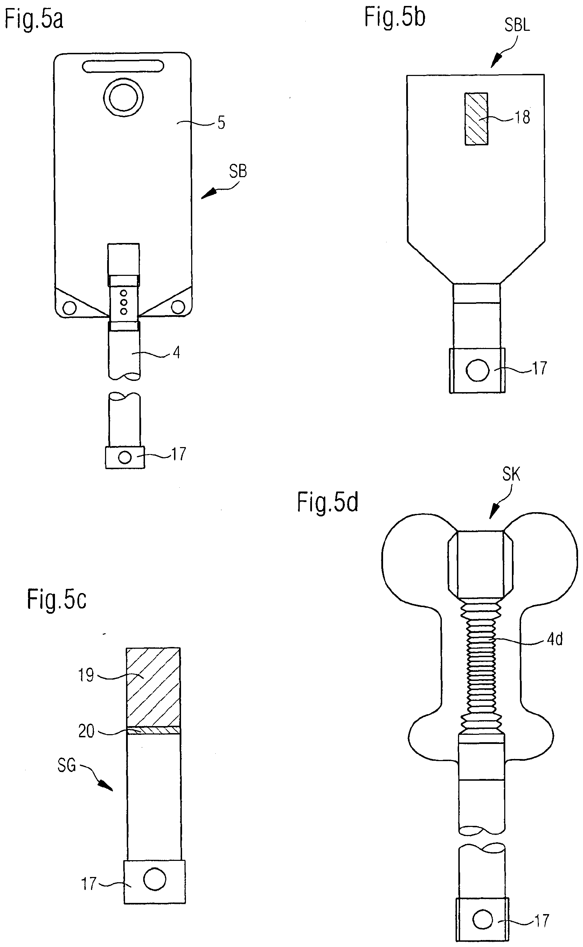

- Abb. 5a: ein Element einer Set-basierten Ausführungsform einer erfindungsgemäßen Vorrichtung, die insbesondere für das kontinuierliche Stuhlmanagement bei dauerhaft immobilen, inkontinenten Patienten mit normaler Stuhlbeschaffenheit ausgelegt ist, wobei im Rahmen der Abbildung ein besonderes Verfahren für die intermittierende, trans-anale Kolonspülung zur Herstellung einer Quasi-Kontinenz von Patienten beschrieben wird, wobei die einzelnen Komponenten dieses Sets in den Fig. 5a bis 5e dargestellt sind: dabei zeigt Abb. 5a einen Spül- und Sammelbeutel mit integrierter Stuhlabscheidungs- und Flussrichtungsfunktion und endständigem Konnektor;
- Abb. 5b: einen leicht ausgeführten, proximal abschließenden Sammel- und Entgasungsbeutel darstellend;
- Abb. 5c: ein mit Gaze-ähnlichem Material gefülltes Abschlusselement mit Entgasungsfunktion darstellend;
- Abb. 5d: eine besondere Ausführungsform einer trans-anal platzierten Kopfeinheit darstellend, mit wellig ausgeformtem, trans-anal verjüngtem, stuhlableitendem Schaftelement;
- Abb. 5e: einen axial steckbaren, flach kollabierbaren Konnektor mit axial wirkender Lock-Funktion darstellend;
- Fig. 6: die einfachste Ausführungsform der Kopfeinheit einer erfindungsgemäßen Vorrichtung;
- Fig. 7: eine andere Ausführungsform der Erfindung, die über ein separates, trans-anal verlaufendes dünnwandiges Rohr- bzw. Schlauchstück verfügt, welches den Trichter mit dem prä-analen Konnektor durchgängig verbindet;
- Fig. 8: eine Kombination von Ankerballon mit einem aus der Ankerballonhülle hervorgehenden trans-analen Segment gemäß Fig. 6, mit dem in Fig. 7 dargestellten, stabilisierenden Rohrstück;

- Fig. 9: eine abgewandelte Ausführungsform der Erfindung, deren transanales Segment über zwei konzentrische Schlauchlagen verfügt;
- Fig. 10: eine wiederum abgewandelte Ausführungsform der Erfindung, welche die zuvor Ausführungsform nach Fig. 9 mit dem in Fig. 8 dargestellten, elastisch verformbaren, sich selbst auf- und ausrichtenden Rohrgeflecht kombiniert;
- Fig. 11: eine weitere Ausführungsform der Erfindung in einer perspektivischen Darstellung; und
- Fig. 12: einen Längsschnitt durch die Fig. 11 entlang der Linie XII - XII.

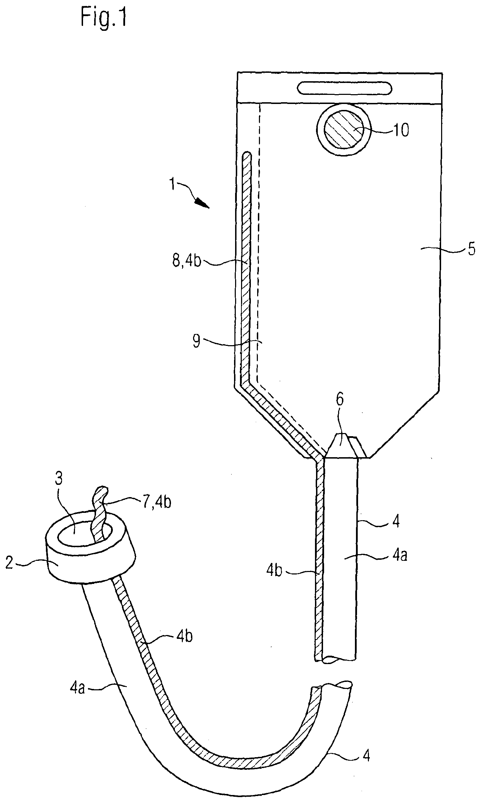

Abb.1 zeigt ein im Rahmen der Erfindung beispielhaftes, einkammeriges Spül-Kathetersystem 1 zur flussrichtungsgetrennten, stuhlabscheidenden Zuleitung von Flüssigkeit in das Rektum bzw. Colon eines Patienten sowie zur Ableitung von mobilisiertem Darminhalt. Am vorderen Ende des Katheters befindet sich ein vorzugsweiser flacher, toroidalförmiger Retentionsballon 2, der eine großlumige zentrale Öffnung 3 mit einem Durchmesser von bevorzugt 15 bis 30 mm für die intra-rektale Aufnahme von flüssigem bzw. fleißfähigem Stuhl ausbildet. An den Ankerballon schließt sich nach proximal ein dünnwandig ausgeführtes, vorzugsweise flach kollabierbares Schlauchfolienelement 4 an, dass zwei parallel verlaufende Lumina integriert und den im Patienten liegenden Teil der Vorrichtung mit dem sich außerhalb des Patienten befindenden Spül- und Sammelbeutel 5 verbindet. Das die Spülflüssigkeit zuleitende Lumen 4b weist einen relativ kleineren Durchmesser auf, der den Fluss der zugeleiteten Spülflüssigkeit reduziert bzw. drosselt, um Irritationen durch zu raschen Einstrom bzw. eine damit einhergehende abrupte Dilatation des Rektums zu vermeiden. Das den Darminhalt ableitende Lumen 4a hingegen ist im Durchmesser derart groß bemessen, dass ein möglichst unbeeinträchtigter Abfluss geformter Stuhlfragmente in den Behälter bzw. Beutel 5 möglich ist. Das besondere funktionelle Prinzip der Vorrichtung beruht auf der Integration flussrichtender und stuhl-abscheidender Elemente bzw. Funktionen, die in Summe verhindern, dass geformte Stuhlanteile, die sich bereits im Beutel befinden, in einer folgenden Einlaufphase zurück in den Darm gespült werden. Hierzu wird im vom Patienten ableitenden Schenkel 4a, vorzugsweise im Übergangsbereich des ableitenden Schenkels zum Beutel, eine ventilartige Einheit 6 als beispielsweise Klappen- oder Lippenventils angebracht sein. Eine entsprechende flussrichtungstrennende Ventilfunktion im zuleitenden Schenkel 4b wird vorzugsweise im Bereich der patientenseitigen Mündung des Schenkels durch ein Funktionselement 7 hergestellt, welches sich beispielsweise als dünnwandiger, leicht kollabierbarer Schlauchstutzen darstellt, der sich bei von innen wirkender Kraft öffnet und bei von außen wirkender Kraft verschließt. Alternativ können die Elemente 6 und 7 auch in anderen Segmenten der Schlauchsysteme oder auch im Behälter selbst verbaut sein.

Zur Trennung bzw. Abscheidung bereits drainierter, flockiger oder geformter Stuhlanteile aus dem Beutelinhalt ist die Vorrichtung mit einem abscheidenden Einlasselement 8 versehen, welches im Beutelinneren bevorzugt bis an den oberen Beutelrand ragt und gewährleistet, dass während der Zuleitungsphasen der Wiedereintrag von bereits abgeleiteten, geformten Stuhlanteilen in den Darm vermieden wird. Das Element 8 kann beispielsweise als netzschlauchartige Struktur oder auch als vielfach perforiertes, siebartiges Rohr gestaltet sein. Zur Fixierung und zum Schutz des Einlasselementes im Beutel kann das Element 8 durch eine ebenfalls perforierte, sieb- oder netzartige Trennfolie 9 innerhalb des Beutels eingehaust sein, deren Perforationen wiederum bevorzugt größer sind als die Perforationen des Elementes, wodurch besonders große, geformte Stuhlfragmente am Vordringen in das das Element aufnehmende Kompartiment gehindert werden. Der Okklusion des Einlasselementes durch Stuhl beim repetitiver Hebe- und Senkeinlauf, wird in der abgebildeten Ausführung durch die Kombination eines innerhalb des Beutels besonders langen, bis an den oberen Beutelrand reichenden Einlasselementes, als auch durch eine dem Einlass der zugeleiteten Flüssigkeit vorgeschalteten, weiteren Filterfunktion vorgebeugt. Der einkammerige Beutel wird durch eine verschließbare Öffnung 10 befüllt.

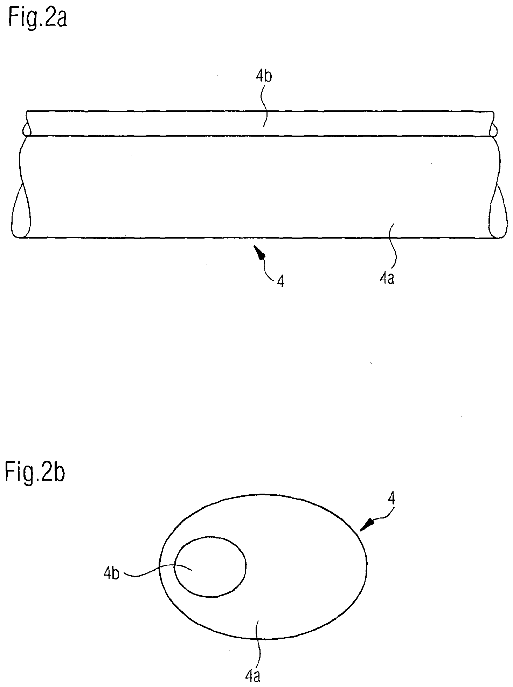

Abb. 2a zeigt ein zwei-lumiges Schlauchelement 4 aus folienartiger, leicht kollabierbarer Schlauchfolie, mit parallelem Verlauf der Einzellumina. Das zuleitende Lumen 4b weist dabei einen runden Querschnitt von 5-10 mm, bevorzugt 6-8 mm auf. Das ableitende Lumen misst im gerundeten Durchmesser 20 bis 35 mm, bevorzugt 20 bis 25 mm. Der Schlauchfolienkörper ist hier durch eine längsverlaufende Separierung, z.B. eine durchgängige Schweißnaht, in zwei separate Lumina aufgeteilt. Das verwendete Schlauchmaterial ist vorzugsweise ein Polurethan der Härte 80A bis 90A und hat eine Wandungsstärke von 150 bis 300 µm, bevorzugt 200 bis 250 µm. Alternativ kann der Schlauch aus PVC, LDPE oder anderem Material mit der Option zur Ausformung ausreichend dünnwandiger, vorzugsweise leicht kollabierbarer Folienschläuche aufgebaut sein. Für die erfindungsgemäße Funktion ist in besonderem Maße die Fähigkeit des Schlauchelementes erforderlich, flach und ohne Ausbildung von stoßenden oder schneidenden Kanten zu kollabieren, dass im Falle eines Aufliegens des Patienten auf dem Schlauch die Entstehung druckbedingter Läsionen vermieden werden kann.

Abb. 2b zeigt ein Schlauchelement 4, wobei die Lumina 4a und 4b zueinander koaxial positioniert sind. Der kleinerlumige, zuleitende, innere Schlauch 4b ist dabei bevorzugt in Relation zum Außenschlauch besonders dünnwandig ausgeführt. Er weist, eine Wandungsstärke von 30 bis 150 µm auf, bevorzugt 50 bis 100 µm. Hierdurch kollabiert das Lumen 4b bereits bei kleiner von außen einwirkender Kraft. Die besondere Kollapsneigung gewährleistet, dass in der Phase der Ausleitung von Darminhalt das abströmende Material den zuleitenden Schlauch komprimiert und so das gesamte ableitende Lumen ohne wesentliche Raumforderung freigibt. Um im Lumen 4b keinen Stauungseffekt während der Ableitung einen raumfordernden Stauungseffekt zu vermeiden, ist die flussrichtende Ventilfunktion im zuleitenden Schenkel bevorzugt am distalen Ende der Drainage, wie beispielhaft in Abb.1 dargestellt, angebracht.

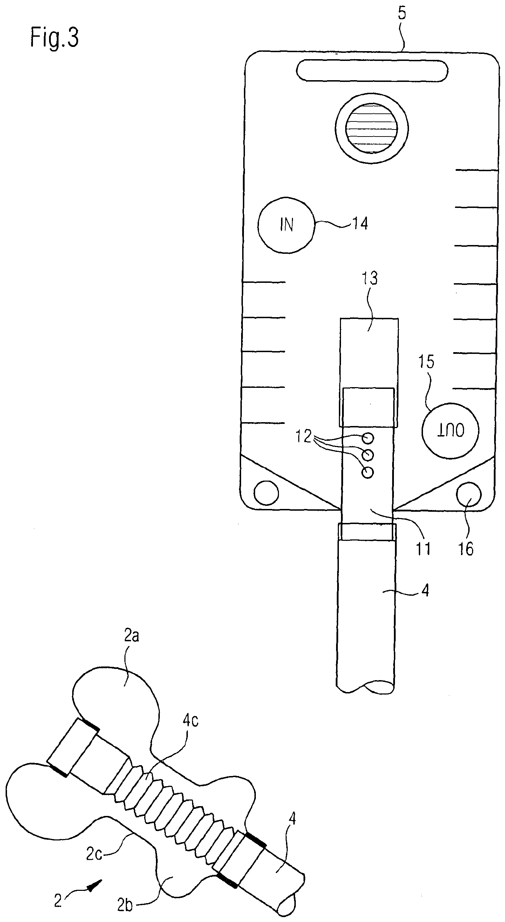

Abb. 3 zeigt einen besonders einfach ausgeführten, erfindungsgemäßen Beutel 5 mit einem bodenseitig in den Beutel eingeschweißten, groß-lumigen Rohrkörper 11, der mit mehreren klein-lumigen Öffnungen 12 versehen ist, durch die aus dem Beutel ausströmen und durch den ab-/zuleitenden Schlauch 4 zum Patienten hingeleitet werden kann, welche aber der Abstrom geformter Anteile aus dem Beutel verhindern. Am innenseitigen Ende des Rohrkörpers ist in der bevorzugten Ausführung dünnwandiges, spontan kollabierbares Schlauchelement 13 angebracht, das eine Ventilfunktion ermöglicht und nur den beutelwärts gerichteten Einstrom von abgeleitetem Material erlaubt, den Ausstrom durch das Lumen des Rohrkörpers aber verhindert. Die abgebildete Bauform gewährleistet die erfindungsgemäße stuhlabscheidende Funktion, wobei der Ein- und Ausstrom in den Beutel bzw. die Zu- und Ableitung, durch ein einziges, gemeinsames Lumen, also nicht voneinander separiert erfolgt. Die Vorrichtung benötigt daher nur eine einzige flussrichtende Komponente bzw. Funktion.

Als weiteres Merkmal kann der Beutel mit einer IN-Skala 14 versehen sein, die das in den Beutel eingefüllte Volumen anzeigt. Ihr gegenübergestellt ist eine OUT-Skala 15, die um 180 Grad gedreht aufgebracht ist, und den das abströmende Material dann misst, wenn der Beutel im Anschluss an die initiale repetitive Spülung und Ableitung kopfüber gewendet und dann dauerhaft als Sammelbeutel verwendet wird. Die Beutel ist im unteren Bereich hierzu mit entsprechenden Halterungen 16 versehen.

Die Abbildung zeigt ferner eine im Rahmen der Erfindung bevorzugt ausgeführte, im Rektum positionierte Kopfeinheit mit einem trans-anal dichtenden Retentionsballon 2, der eine rektal platzierte Erweiterung 2a, eine prä-anale Erweiterung 2b und eine trans-anal verjüngte Portion 2c aufweist, sowie über einen besonderen, den Ballon tragenden Schaftschlauch 4c verfügt, der bei anal anlastender Kraft in elastischer Weise radial kollabiert und sich bei nachlassender Kraft spontan aufrichtet, sowie sich bei axialer Kraftwirkung ziehharmonikaartig staucht und so Perforationen des Darms vorbeugt.

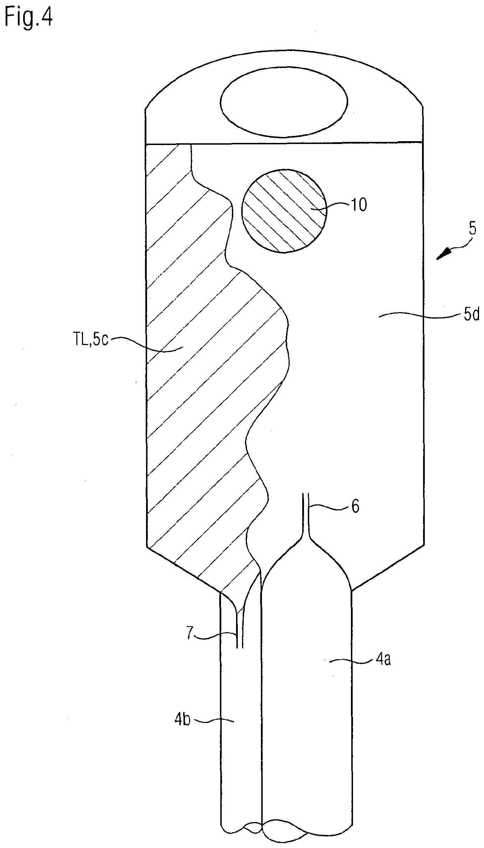

Abb. 4 zeigt ein vollständig getrenntes, zwei-kammeriges Beutel- bzw. Behältersystem 5, welches eine Vermischung des einströmenden und des ausströmenden Materials vollständig verhindert. Die beiden Kompartimente 5c (Einlauf-Kompartiment) und 5d (Sammelkompartiment) sind durch eine Trennlage TL vollständig räumlich separiert. Das Kompartiment 5c geht an seinem unteren Ende in das den Einlauf zuleitende Lumen 4b über. Das sammelnde Kompartiment 5d ist hier vollständig geschlossen und kann nicht entleert werden. Nur das Einlauf-Kompartiment verfügt über eine Füllöffnung. Die Ventilfunktionen 6 und 7 sind hier jeweils durch lippenartig oder auch schnabelartig gestaltete Ventile gewährleistet.

In funktioneller Überleitung von einem zwei-kammerigen System in ein einkammeriges System kann die Trennlage TL als netzartiges, fliesartiges oder siebartig perforiertes Element ausgebildet sein und somit den Übertritt von geformten Stuhlanteilen aus dem Kompartiment 5d in das Kompartiment 5c verhindern.

Abb. 5a bis 5e zeigt ein Spülsystem zur Herstellung bzw. Aufrechterhaltung eines quasi-kontinenten Zustandes eines Patienten durch weitestgehende Ausräumung bzw. Lavage von Darminhalt, als eine insgesamt Set-basierte Ausführungsform S, welche als das Set umfassende Komponenten zum einen eine trans-anal dichtende Kopfeinheit SK aufweist, die bevorzugt nach Abb. 3 ausgeführt ist, und mit einem hantelförmigem, trans-anal dichtenden Ballon und einem trans-anal positionierten, atraumatisch falt- bzw. stauchbaren Schaftschlauch ausgestattet ist, an den sich ein elastisch faltbarer zu- und ableitender Schaftschlauch 4 anschließt, der wiederum in relativer Nähe zur Kopfeinheit, bevorzugt in einem Abstand von ca. 20 cm vom Anus, in ein endständiges Konnektorelement 17 übergeht.

An dieses Konnektorelement wird im zyklischen Wechsel, als weitere Set-Komponente ein Beutelsystem 5, SB für die kombinierte hoch-volumige Irrigation und nachfolgende Drainage, oder alternativ ein einfacher, leicht ausgeführter Auffangbeutel SLB für die Aufnahme von abgehendem Sekret mit Entgasungsfunktion nach Abb. 5b, oder ein Gaze befülltes, stutzenförmiges Abschlusselement SG nach Abb. 5c, zur primären Entgasung des Abdomens angeschlossen.

Die Konnektion der dauerhaft trans-anal im Patienten verbleibenden Kopfeinheit zu den jeweils angeschlossenen Komponenten SB, SLB und SG, wird bevorzugt durch ein in der Bauart vorzugsweise Abb. 5d entsprechendes Konnektorelement 17 hergestellt, wobei dieses derart gestaltet ist, dass es bei einem eventuellen Aufliegen des Patienten auf dem Konnektor elastisch reversibel kollabiert bzw. eine möglichst abgeflachte, bandartige Struktur übergeht und somit Druckstellen vermieden werden können.

Die besondere bauliche Auslegung der hier als Set von Produktkomponenten beschriebenen Vorrichtung ist primär für immobile Patienten konzipiert, die kontinuierlich oder über sporadische, zum Teil länger anhaltende Intervalle stuhlinkontinent sind und überwiegend Stuhl von normaler, geformter Konsistenz produzieren. Die mit dem System mögliche, zuverlässig trans-anal dichtende, besonders hoch-volumig durchgeführte Lavage des Colon, vermeidet das sich Stuhl im Colon formen und festigen kann, und hält das zum Rektum hin absteigenden deszendierende Colon weitgehend von Stuhl frei, so dass im idealen Falle kein Stuhl in das Rektum vordingen kann, und so Quasi-Kontinenz ermöglicht wird.

Das erfindungsgemäße Verfahren zur Herstellung einer Quasi-Kontinenz kombiniert folgende Funktionsmerkmale des Sets:
- eine im Patienten dauerhaft, kontinuierlich platzierte Kopfeinheit SK, die zum einen eine Dichtung des Anus gegen aus dem Rektum austretendes Sekret oder Darminhalt ermöglicht, und zum anderen einen permanenten groß-lumigen Zugang für eine intermittierende, hoch-volumige, repetitive Spülung und Drainage im Sinne eines Schwenkeinlaufs schafft;
- einen kombinierten Spül- und Sammelbeutel SB, der im etwa zweitägigen Rhythmus an die dauerhaft platzierte Kopfeinheit angeschlossen wird, und einen repetitiven Schwenkeinlauf ermöglicht, wobei die dem Patienten zugeführte Flüssigkeit vorzugsweise von bereits in den Beutel abgeleiteten, geformten Stuhlanteilen abgeschieden wird, und der Beutel nachfolgend, im Anschluss an die Phase der Lavage, verzögert, in Fraktionen ausgeschiedenen, mobilisierten Darminhalt aufnimmt bzw. eine geschlossene, leckagefreie, mehrstündige, der Drainage nachfolgende Stuhldrainage erlaubt;
- wahlweise einen leicht ausgeführten, kleinen Abschlussbeutel SLB mit sekretaufnehmender und entgasender Funktion, oder einen mit leichter Gaze befüllten Abschlusspropfen SG aufweist, die jeweils in den Phasen zwischen den Auswaschungen vor austretenden Sekreten und Darminhalt schützen bzw. eine kontinuierliche Entgasung ermöglichen,

Das erfindungsgemäße Verfahren zur Erreichung einer Quasi-Kontinenz durch hoch-volumige Auswaschung von Darminhalt stellt sich wie folgt dar:
- nach der trans-analen Positionierung der großlumig drainierenden, trans-anal dichtenden Kopfeinheit wird zur Durchführung eines initialen Lavagezyklus ein kombinierter Spül-und Sammelbeutel mit optionaler Stuhlabscheidungsfunktion bzw. ein entsprechend funktionell gestaltetes Spül- und Sammelgefäß an das terminale Konnektorstück der Kopfeinheit angeschlossen;
- im Anschluss an die Irrigation verbleibt der Beutel/das Gefäß dann zur Sammlung nachfolgenden, verzögert ausgeschiedenen Stuhls am Patienten bzw. an der Kopfeinheit, bis keine weitere Ausscheidung von mobilisiertem Stuhl mehr stattfindet;
- sind keine Stuhlabgänge mehr erkennbar, wird der Spül- und Sammelbeutel durch einen leichten Entgasungsbeutel oder einen Entgasungsstutzen ersetzt, welcher jeweils bis zum nächsten Irrigationszyklus mit der Kopfeinheit verbunden bleibt;
- bei intermittierender Lavage des Colon kann die Rektumampulle des Patienten so für ca. 48 Stunden in einem weitgehend stuhlfreien Zustand gehalten werden.

Abb. 5a zeigt einen Sekretsammelbeutel SB nach Abb.3, dessen zu- und ableitende Schlaucheinheit 4 einen endständigen Anschlusskonus 17 aufweist.

Abb. 5b zeigt und einem Beutel SLB mit einem in die Beutelwandung integrierten Entgasungs- bzw. Filterelement 18.

Abb. 5c zeigt einen entlüftenden, mit Gaze 19 befüllten Abschlusspfropfen SG, der primär Darmgas ablässt und optional auch eine sekretaufsaugende Funktion hat, oder die endständige Gazeportion 19 durch eine gasdurchlässige, aber flüssigkeitsundurchlässige Lage 20 vor ablaufendem Sekret oder Stuhl geschützt ist.

Abb. 5d zeigt eine Kopfeinheit SK, deren Bauart vorzugsweise Abb.3 entspricht, deren trans-anal stuhlableitende Schaftkomponente 4d aber bevorzugt eine zum distalen und proximalen Ende des Schaftes relative, mittige Verjüngung von ca. 15 bis 17 mm auf ca. 10 bis 12 mm Durchmesser aufweist, die eine besonders atraumatische, langfristige trans-anale Positionierung der Vorrichtung erlaubt. Insbesondere kann der den Analkanal aufnehmende, so taillierte Schaftschlauch durch ein welliges Profil verstärkt bzw. seine elastischen Faltungs- und Aufrichtungseigenschaften optimiert werden. Die Amplitude und/oder Breite der Wellung verkleinern sich dabei im Übergang vom jeweils endständigen großen Lumen zum mittigen kleinen Lumen hin. Die Schaftkomponente 4d soll im Analkanal ein möglichst flaches, dennoch leicht biegbares und spontan falt- und knickbares Profil entwickeln

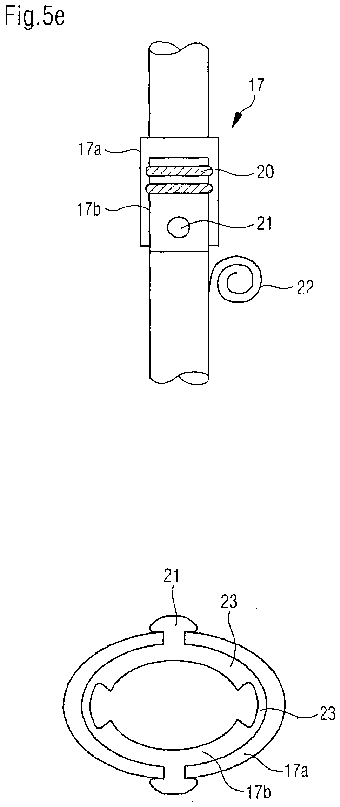

Abb. 5e zeigt ein besonderes Konnektorelement 17, dass über zwei ineinander, kongruent steckbare, optional leicht konische Zylinder 17a und 17b, aus bei moderater Krafteinwirkung von außen elastisch verformbaren bzw. flach kollabierbaren, und sich bei nachlassender Kraft elastisch aufrichtenden, körperfreundlich weichem Material, wie z.B. spritzgegossenes Polyurethan der Shore Härte 40A-80A, vorzugsweise 60 bis 70A verfügt. Die Zylinder 17a und/oder 17b tragen auf der Kontaktfläche aufgebracht ringförmige Dichtungselemente oder Dichtungslippen 20, die der elastischen Verformungsmechanik der Zylinder folgen bzw. dieser entsprechen. Der konvexe Radius der Dichtungslippen rastet vorzugsweise im Gegenstück in entsprechend geformte, aufnehmende Ringnuten, um neben der Dichtung eine axiale Fixierung der Zylinder zueinander herzustellen und deren axiale Diskonnektion zu vermeiden. Die Dichtungslippen und Nuten sind derart gestaltet, dass sie sich in möglichst kleinen Übergangsradien vom Zylinder erheben oder einsenken und somit einen möglichst senkrechten Winkel ausbilden, was insbesondere der axialen Diskonnektion entgegenwirkt. Einer axialen Diskonnektion durch stärkere, auf die Konnektion axial anlastende Zugkraft kann ferner durch zapfenartige, krallenartige oder klebend heftende oder auch mit Klettband ausgestattete Strukturen bzw. Elemente 21 vorgebeugt werden, wobei die jeweiligen Strukturen beide Teile des Konus miteinander vorzugsweise rastend verzapfen oder verzahnen, oder durch abpeelbaren bzw. reversibel lösbaren Kleber oder auch durch Verklettung verbinden, oder beispielsweise auch durch einen bajonettartig rastenden Mechanismus sichern. Um die konnektierenden Zylinder noch weicher bzw. möglichst atraumatisch für den Patienten zu gestalten, kann die konnektierende Baugruppe 17 von einer weichen, vorzugsweise gelartigen bzw. gelelastischen, strumpfartigen Hülle 22 umscheidet sein, die z.B. auf einer Konnektorhälfte fest aufgebracht bzw. auf dieser strumpfartig aufgerollt ist, und von dort aus auf die gegenüberliegende Konnektoren Oberfläche abgerollt werden kann, und mit einer gewissen Spannung dichtend und/oder auch heftend bzw. reversible klebend auf dieser aufliegt. Die beiden ineinander gesteckten Konnektorhälften können jeweils in bestimmten Umfangbereichen 23 abweichende Wandstärken haben, wobei bevorzugt Wandstärkenbereiche gleicher Wandstärke einander gegenüberliegen. Diese bestimmte Weise der Wandstärkenverteilung ermöglicht einen präformierten Kollaps des Konnektors, so dass dessen Profil bei Druck von außen leichter knickt bzw. flach kollabiert. Die Elemente 21 sind dabei bevorzugt in den Bereichen mit der relativ stärker ausgeführten Wandung angebracht.

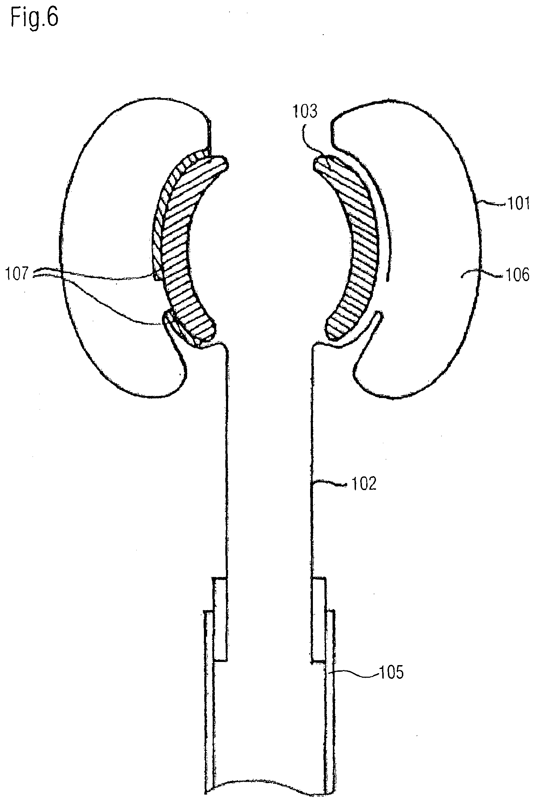

Bei dem in Fig. 6 dargestellten Kopfstück einer Vorrichtung zur Durchführung einer lavage-artigen, repetitiven Zu- und Ableitung einer spülenden, reinigenden oder sonstigen therapeutisch wirksamen Substanz, insbesondere im Rahmen einer groß-lumig spülenden bzw. ableitenden Reinigung des Colons eines Patienten, kann ein intra-rektaler Ankerballon die Form eines intra-rektalen Segmentes 101 aufweisen und zusammen mit einem transanalen Zwischensegment 102 aus einem einzigen gemeinsamen Schlauch-Rohling 108 gefertigt sein, wobei das trans-anale Segment 102 der ausgeformten Ballonfolie 108 lediglich eine ein-lagige Wandung aufweisen kann. Intra-rektales und transanales Segment 101, 102 der Ballonfolie 108 bilden in der dargestellten Vorrichtungsvariante zwar eine strukturelle Einheit, weisen im montierten und auf/an einem Trichterelement 103 fixierten Zustand jedoch eine funktionelle Trennung auf.

Beide Segmente 101, 102 der Folie 108 können durch ein von distal her in den Ankerballon 101 eingesetztes Trichterelement 103 voneinander separiert bzw. dichtend getrennt sein. Durch die dauerhafte Verbindung von Ballonfolie 108 in dem Bereich des intra-rektalen Ankerballonsegments 101 mit dem Trichterelement 103 entsteht ein befüllbares Kompartiment 106, welches sich nach Art einer Manschette um die seitlichen Partien des Trichterelements 103 entfalten kann. Die Verbindung kann durch besonders gestaltete, und im Folgenden näher beschriebene Präformationen 107 der Trichteroberfläche und/oder der Ballonfolie 108 vereinfacht bzw. verbessert werden.

Dem trans-analen Segment 102 schließt sich nach proximal hin ein die Kopfeinheit 101, 102 mit einem Spül- und Sammelbeutel oder mit einer Sammel- und Entgasungseinheit verbindende Schlaucheinheit 105 an.

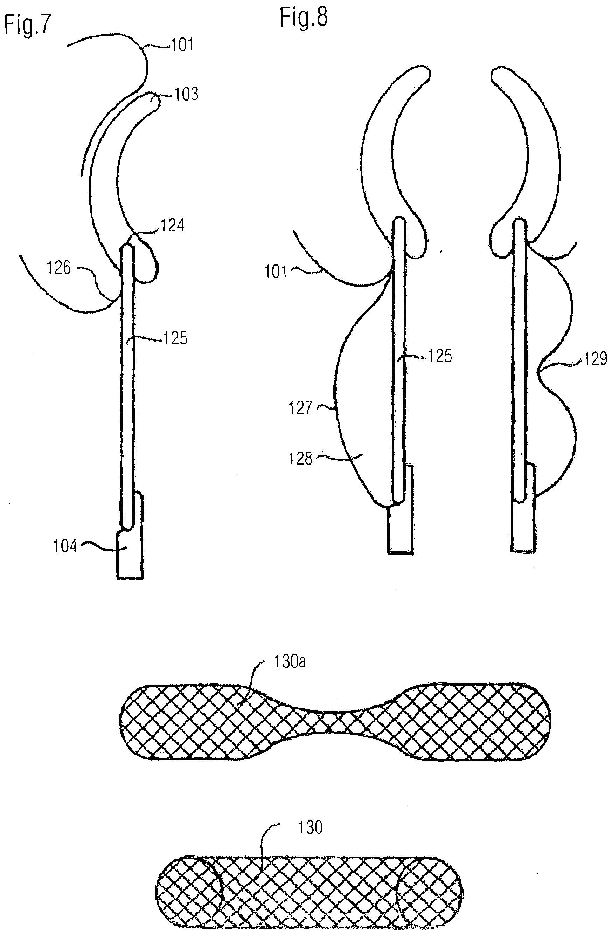

Die in Fig. 7 dargestellte Ausführungsform eines Kopfstücks für eine erfindungsgemäße Vorrichtung verfügt anstatt über ein aus dem Ankerballonsegment 101 hervorgehendes, nach proximal verlängertes Ballonende 110, wie z.B. in Fig. 6 dargestellt, über ein separates, trans-anal verlaufendes, dünnwandiges Rohr- oder Schlauchstück 125, welches den Trichter 103 mit dem prä-analen Verbindungselement oder -schlauch 104 durchgängig verbindet. Hierbei handelt es sich um ein Element aus einem elastischen Werkstoff mit hoher Rückstellkraft, welches trotz möglichst dünnwandiger Ausführung bestrebt ist, sich bei einer radialen Verformung selbständig aufzurichten und/oder bei einer Torsion um seine Längsachse selbständig zu entwinden, und so spontan in seinen spannungsarmen lumenoffenen Ausgangszustand zurückzukehren.

Die Fig. 7 zeigt beispielhaft, wie die Verbindung zwischen dem Rohr- oder Schlauchstück 125 und dem Trichterelement 103 durch eine präformierte Ringnut 124 am proximalen Trichterende gestaltet werden kann. Das proximale Ende 126 des intrarektalen Ballonhüllensegments 101 kann dabei derart über das distale Ende des Rohr- oder Schlauchstücks 125 gelegt, gestülpt oder in sonstiger Weise geführt werden, dass dieses durch das Einstecken des distalen Endes des trans-analen Segmentes 101 in die Ringnut 124 dauerhaft dichtend mit dem Trichterelement 103 verbunden wird.

Fig. 8 zeigt eine Kombination von intra-rektalem Ankerballon 101 mit einem aus der Ankerballonhülle 108 hervorgehenden trans-analen Segment 102 wie in Fig. 6 dargestellt, mit dem in Fig. 7 beschriebenen stabilisierenden Rohrstück 125. Das trans-anale Segment 102 bildet dabei ein separat befüllbares Kompartiment 128 aus, welches zwischen dem innen liegendem Rohr- oder Schlauchstück 125 und der äußeren nach proximal verlängerten Anker-Ballonhülle 127 entsteht. Bei Befüllung dieses Kompartiments 128 kann, abhängig von der Volumendehnbarkeit der Hülle bzw. ihres bei der Herstellung ausgeformten Durchmessers, durch radiale Ausdehnung der äußeren Hülle 127 die Dichtung zum Anus hin verbessert werden. Bei forcierter Befüllung kann darüber hinaus das Drainagelumen der Kopfeinheit zum Zentrum hin verengt und annähernd verschlossen werden. Alternativ zur Ausformung der Hülle 127 aus dem proximalen Ausläufer des intra-rektalen Ankerballonsegments 101 kann für die Hülle 127 auch ein separat hergestellter Ballon- oder Folienschlauchkörper zur Anwendung gelangen. Die das Kompartiment 128 nach außen hin begrenzende Hülle 127 kann darüber hinaus hantel- oder sanduhrförmig präformiert sein. Eine dabei bevorzugt etwa mittig angeordnete Taillierung 129 ist dabei derart ausgeformt, dass sie die Strukturen des Analkanals aufnimmt. Wird das trans-anale Kompartiment 128 mit Druck beaufschlagt, wie dies z.B. bei der Instillation der Irrigationsflüssigkeit erfolgt, gewährleistet die beschriebene Präformation 129 zum einen eine sichere Verankerung des Kopfteils 101 der Vorrichtung, zum anderen trägt sie zur Dichtung gegen die von rektal her anlastende Spülflüssigkeit bei.

Zur Fixierung und zum dichtenden Abschluss der für diese Ausführungsform erforderlichen Komponenten im Übergangsbereich zwischen intra-rektalem und trans-analem Kompartiment 106, 127 können die zuvor beschriebenen Techniken verwendet werden.

Analog kann das bevorzugt aus dem intra-rektalen Ankerballonsegment 101 hervorgehende Folienschlauchsegment 127 mit einem radial verformbaren, sich den trans-analen Konturen unter leichter Spannung anschmiegenden Rohrgeflecht 130 kombiniert werden. Das Rohrgeflecht 130 weist im nichtverformten, spannungsarmen Ausgangszustand ein offenes Lumen von ca. 2 bis 3 cm auf. Die sich bei Lumen-Einengung entwickelnde radial gerichtete Rückstellkraft auf den Anus ist dabei gering und schließt die Entstehung von Druckulcera aus. Kommt es beim Defäkationsreflex zu einer Tonusreduktion des analen Schließmuskels, folgt das Rohrgeflecht 130 dem sich dann öffnenden trans-analen Kanal und erleichtert so den Stuhlabfluß. Das Rohrgeflecht 130 verbindet den intra-rektalen Trichter 103 mit einem prä-analen Verbindungselement oder -schlauch 104 vorzugsweise durchgehend. Es ist bevorzugt im Inneren des Schlauchsegmentes angeordnet, kann jedoch alternativ auch über dessen äußere Oberfläche verlaufen. Im radial eingeengten Zustand 130a weist das vorzugsweise aus starren, biegbar verformbaren Filamenten aufgebaute Geflecht 130 einen deutlichen Längenzuwachs auf, der durch eine entsprechende Längenvorgabe des umgebenden trans-analen Folienschlauches 127 berücksichtigt ist.

Die Montage der anteiligen Komponenten folgt den zuvor beschriebenen Techniken.

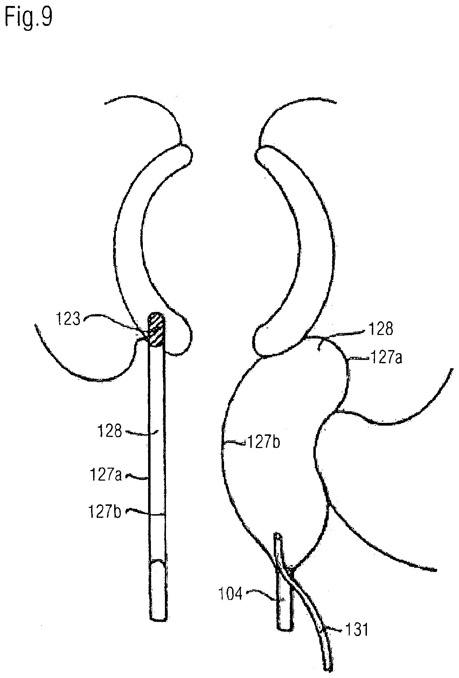

Fig. 9 stellt eine Ausführungsform dar, deren trans-anales Segment über zwei konzentrische Schlauchlagen 127a und 127b verfügt. Diese können, wie zuvor beschrieben, aus den verlängerten Enden des intra-rektalen Ballonsegments 101 gefertigt sein. In der bevorzugten Ausführungsform geht die innere Schlauchlage 127b aus der proximalen Verlängerung der intra-rektalen Ballonsphäre 109 hervor. Die äußere Schlauchlage 127a wird bevorzugt als separates dünnwandiges, zylindrisch oder auch dem Analkanal kongruent präformiert ausgeformtes Folienschlauchelement gestaltet.

Alternativ können die beiden Schlauchlagen 127a, 127b auch aus jeweils separat gefertigten Schlauchfolien bestehen.

Der zwischen den beiden Schlauchlagen 127a, 127b entstehende Raum 128 kann über eine über ein Verbindungselement oder -schlauch 104 geführte Zuleitung 131 von extrakorporal mit einem Medium partiell oder vollständig befüllt oder auch vollständig evakuiert werden.

Im evakuierten Zustand liegen die beiden Schlauchlagen 127a, 127b einander fest an und verhalten sich quasi wie eine einlagige Wandung. Im befüllten Zustand trennen sich die beiden Lagen 127a, 127b voneinander, das Drainagelumen ist maximal geöffnet.

Im prall befüllten Zustand dehnt sich die innere Lage 127b zum Zentrum des Drainagelumens hin aus und verschließt dieses flüssigkeitsdicht. Die äußere Lage 127a hingegen dehnt sich zur analen Wandung hin aus und schmiegt sich dieser, der jeweiligen Anatomie folgend, gegen diese ebenfalls dichtend an.

Bei teilweiser Befüllung bzw. Befüllung mit wenigen Millilitern Füllmedium trennen sich die beiden Lagen 127a, 127b. Die beiden dünnwandigen Folienlagen 127a, 127b gleiten, durch das Füllmedium quasi gelagert, frei verschieblich zueinander. Durch das freie Spiel und Gleiten der Folienlagen 127a, 127b können im besonders sensiblen Bereich des Analkanals Läsionen, wie sie durch statisch anliegende, wenig dynamische Folien bekannt sind, besser vermieden werden.

Die Verbindung der anteiligen Komponenten mit dem proximalen Trichterende kann auf der Basis bzw. mittels eines ringförmigen Trägers 123 erfolgen, der in eine ringförmige Präformation 124 am proximalen Trichterrand eingesetzt wird. Die innere Lage 127b kann dabei wie in Fig. 6c über den Ring 123 geführt und mit dem Trichter 103 verbunden werden. Die äußere Lage 127a kann in einem vorausgehenden Montageschritt an ihrem distalen Ende auf dem Ring 123 aufgeklebt werden.

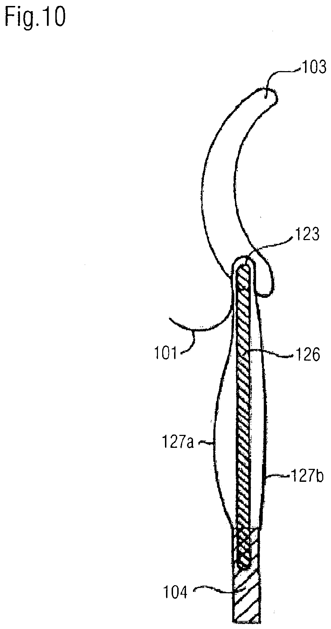

Fig. 10 kombiniert die zuvor beschriebene Ausführung mit dem in Fig. 8 beschriebenen elastisch verformbaren, sich selbst auf und ausrichtenden Rohrgeflecht 130. Alternativ zu einem Rohrgeflecht 130 kann das zuvor beschriebene Schlauch- oder Rohrelement 125 verwendet werden.

Die zuvor beschriebenen Elemente 125, 130 werden bevorzugt zwischen den beiden Folienlagen 127a und 127b, den Trichter 103 mit einem Verbindungselement oder -schlauch 104 verbindend, verbaut.

Bei allen Ausführungsformen gemäß Fig. 6 bis Fig. 10 kann sich das Trichterelement 103 auch innerhalb des intra-rektalen Kompartiments 106 befinden, also zwischen der äußeren Folienlage 109 und der inneren Lage bzw. Loge 111 des intrarektalen Ankerballonsegments 101.

Alle diese Kopfteile 101, 102 nach einer der Figuren 6 bis 10 sind über ein Schlauchfolienelement 4 mit einem Spül- und Sammelbeutel 5 nach einer der Abbildungen 1 bis 5 koppelbar. Auch kann sich ein Element mit einer flussrichtenden Funktion im Bereich jedes dieser Kopfteile 101, 102 befinden, bevorzugt in einem für die Zuleitung von Spül- oder Irrigationsflüssigkeit zu dem Patienten vorgesehenen Schlauchelement.

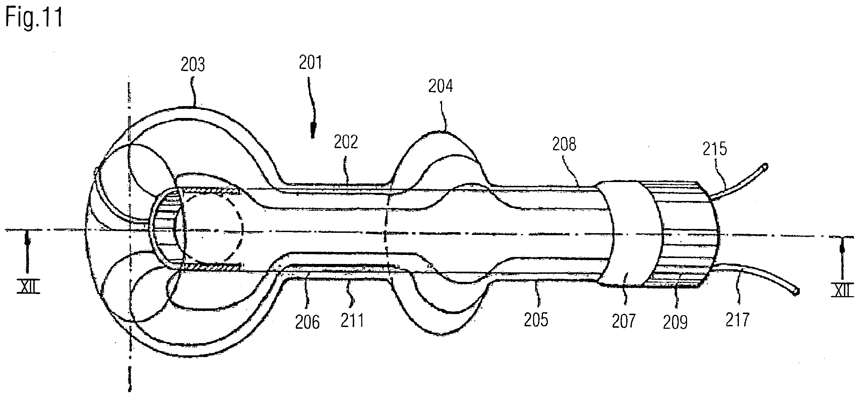

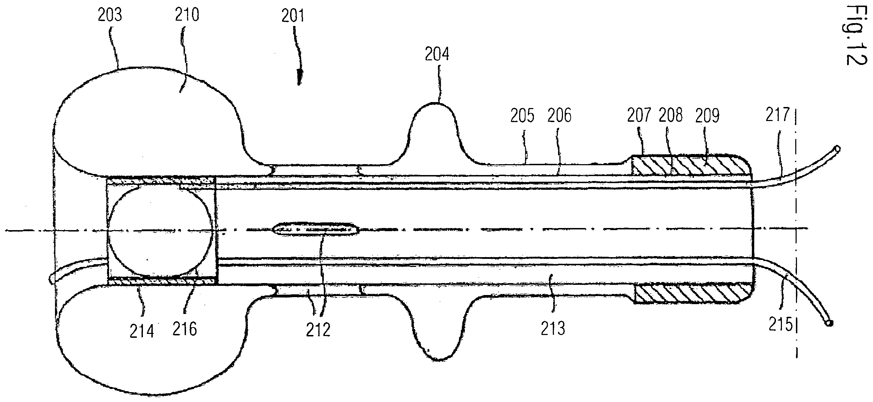

In den Figuren 11 und 12 ist eine weitere Kopfeinheit für eine erfindungsgemäße Vorrichtung 201 zur Durchführung einer lavage-artigen, repetitiven Zu- und Ableitung einer spülenden, reinigenden oder sonstigen therapeutisch wirksamen Substanz, insbesondere im Rahmen einer groß-lumig spülenden bzw. ableitenden Reinigung des Colons eines Patienten, dargestellt, die ebenfalls über ein Schlauchfolienelement 4 mit einem Spül- und Sammelbeutel 5 nach einer der Abbildungen 1 bis 5 koppelbar ist. Auch dabei kann sich ein Element mit einer flussrichtenden Funktion im Bereich jedes Kopfteils 201 befinden, bevorzugt in einem für die Zuleitung von Spül- oder Irrigationsflüssigkeit zu dem Patienten vorgesehenen Schlauchelement.

Die Vorrichtung 201 dient zum Spülen und/oder Verschließen eines natürlichen oder künstlichen Darmausgangs. Sie umfaßt einen präformierten und in sich zurückgestülpten Schlauch 202 aus einem dünnwandigen Material, bspw. aus Polyurethan mit einem Shore-Härtegrad A 90 und einer Wandstärke von weniger als 25 µm.

Durch die Präformierung hat der Schlauch 202, dessen ursprünglicher Durchmesser etwa zwischen 15 mm und 30 mm liegt, zwei radiale Erweiterungen 203, 204 erhalten. Die eine, vorzugsweise größere Erweiterung 203 befindet sich etwa in der Mitte des Schlauchs, die nach der Umstülpung das patientenproximale Ende der Vorrichtung 201 bildet. Die andere Präformierung 204 befindet sich etwa in der Mitte des nach dem Umstülpen außen liegenden Schlauchabschnitts 205, während der innen liegende Schlauchabschnitt 206 keine derartige Erweiterung aufweist, sondern einen gleichbleibenden Querschnitt hat.

Eines oder beide freien Enden 207, 208 der beiden Schlauchabschnitte 205, 206 können mit einem beispielsweise hülsenförmigen Konnektorstück 209 verbunden sein. Das Konnektorstück 209 kann - insbesondere im Bereich seines patientendistalen Endes - ein Innen- und/oder Außengewinde aufweisen zum Anschluß verschiedener medizinischer Apparaturen, bspw. eines Spül- und Sammelbeutels 5, einer extrakorporalen Entgasungseinheit od. dgl.

Vorzugsweise ist die Außenlage 205 des Ballons 202 an der Außenseite des Konnektorstücks 209 festgeklebt, die Innenlage 206 des Ballons 202 kann an dessen Innenseite angeklebt sein. Dadurch ist der Hohlraum 210 zwischen der Innen- und Außenlage 205, 206 des Ballons 202 luftdicht abgeschlossen; lediglich im Bereich des Konnektorstücks 209 befindet sich eine in der Zeichnung nicht dargestellte Verbindung nach außen, an welche eine Quelle mit einem mit Druck beaufschlagbaren Medium anschließbar ist, um den Ballon 202 zu entfalten. Anstelle eines hülsenförmigen Konnektorstücks 209 können die beiden Ballonlagen 205, 206 auch bspw. unmittelbar aneinander befestigt sein, bspw. zusammengeklebt.

Durch die vergleichsweise große Materialhärte des Ballons 202 ist dieser nur geringfügig elastisch und nimmt in inflatiertem Zustand seine in Fig. 11 erkennbare, durch die Präformierung vorgegebene Gestalt an. Diese umfaßt eine etwa zylindrische Grundgestalt mit einer etwa kugelförmigen Erweiterung 203 an dem patientenproximalen, dem Konnektorstück 209 oder einem sonstigen, dortigen Verschluß gegenüber liegenden Ende und mit einer ring- oder scheibenförmigen Erweiterung 204 etwa in der Mitte zwischen den beiden Enden 203, 209.

Die kugelförmige Erweiterung 203 wird in deflatiertem Zustand in dem Rektum eines Patienten plaziert (indorektaler Abschnitt), während der sich anschließende, zylindrische Abschnitt 211 bis zu der ring- oder scheibenförmigen Erweiterung 204 durch den Analkanal nach außen führt (transrektaler Bereich), die patientendistale Erweiterung 204 liegt in der Analfalte.

In dem transrektalen Bereich 211 zwischen den beiden Erweiterungen 203, 204 sind die beiden Lagen des Ballons 202, nämlich die Außenlage 205 und die Innenlage 206, miteinander verbunden, vorzugsweise durch Verschweißungen 212 oder Verklebungen. Es kann sich hierbei um punkt-, linien- oder flächenförmige Verschweißungen handeln. Bei der dargestellten Ausführungsform sind vier in axialer Richtung verlaufende Schweißlinien 212 vorgesehen, die jeweils um etwa gleiche Umfangswinkel gegeneinander versetzt sind. Dank dieser Schweißverbindungen 212 kann das innere Lumen 213 innerhalb der Innenlage 206 des Ballons 202 leichter geöffnet werden, wenn der Darm entleert werden soll.

Im Bereich der patientenproximalen Erweiterung 203 wird das innere Lumen 213 von einer Versteifungshülse 214 offengehalten, deren Länge vorzugsweise gleich oder kleiner ist als die axiale Erstreckung der radialen, indorektalen Erweiterung 203 des Ballons 202. Die Materialhärte der Versteifungshülse 214 ist vorzugsweise gleich oder kleiner als die Materialhärte des Ballons; ihre Steifigkeit erhält die Hülse 214 aus ihrer erhöhten Wandstärke. Bevorzugt kann für die Hülse 214 dasselbe Material verwendet werden wie für den Schlauch bzw. Ballon 202. Dadurch erleichtert sich die Fixierung der Hülse 214 innerhalb des inneren Lumens 213, insbesondere durch Verklebung mit der Innenlage 206 des Ballons 202, wobei solchenfalls ein das betreffende Material anlösendes Mittel als Klebstoff bzw. zur Verschweißung verwendet werden kann.

Vorzugsweise innerhalb des inneren Lumens 213 erstreckt sich ein Schlauch 215 von dem patientenproximalen Ende 203 bis jenseits des Konnektorstücks 209. Durch diesen Schlauch 215, der an dem Ballon 202 - vorzugsweise an dessen Innenlage 206 - fixiert sein kann, bspw. durch Verkleben, läßt sich ein Spülmedium in den Darm des Patienten einleiten. Damit solchenfalls keine Bakterien aus dem transrektalen Bereich 211 oder aus dem Bereich jenseits der transrektalen Erweiterung 204 in den Darm geschwemmt werden können, befindet sich die Öffnung des Schlauchs 215 an dem vordersten Bereich des indorektalen Endes 203 der Vorrichtung 201. Der Schlauch 215 kann innen durch die Versteifungshülse 214 geführt sein oder zwischen derselben und der Innenlage 206 hindurch.

Um während eines Einlaufs das zentrale Lumen 213 optimal versperren zu können, ist weiterhin ein Okklusionsballon 216 vorgesehen. Dieser sitzt bevorzugt innerhalb der Versteifungshülse 214 und hat bei der Ausführungsform nach den Fig. 11 und 12 eine kugelförmige Präformierung mit einem Durchmesser, der etwas größer ist als der Durchmesser der Versteifungshülse 214. Dadurch verschließt der inflatierte Okklusionsballon 216 das zentrale Lumen 213 vollständig und liegt unter Druck sowie unter geringer Verformung rundum an der Innenseite der Versteifungshülse 214 an, wodurch dieselbe verschlossen wird.

An dem Okklusionsballon 216 mündet ein weiterer Schlauch 217, mit dem ein vorzugsweise flüssiges Druckmedium in den Okklusionsballon 216 geleitet werden kann, um das Lumen 213 im Bereich der Versteifungshülse 214 zu verschließen. Der Schlauch 217 ist an der Innenseite der Ballon-Innenlage 206 bis durch das Konnektorstück 209 hindurch geführt und somit von außen zugänglich. Etwa im Bereich der Mündung des Schlauchs 217 in den Okklusionsballon 216 ist letzterer an der Versteifungshülse 214 fixiert, bspw. etwa punktuell angeklebt.

An der jeweiligen Kopfeinheit 2, 101, 102, 201, 201' kann jeweils ein kurzes Schlauchstück angeformt sein, welches dann in einen Schlauch 4 übergehen oder münden kann, woran dann proximal ein Konnektor 17 zum wahlweisen Anschluss eines Spül- und Sammelbeutels 5 oder einer Sammel- und Entgasungseinheit oder eines weiteren, peripheren Elements.

Mit den Figuren 6 bis 12 soll dokumentiert wrden, dass das erfindungsgemäße System und insbesondere auch die erfindungsgemäße Vorrichtung mit verschiedenen Kopfeinheiten verwendet werden kann. Während in Abb. 1 eine einfache, vorzugsweise intrarektal platzierbare Kopfeinheit verwendet werden kann, die ähnlich wie Fig. 6 nur aus einem einzigen Ballonkompartiment besteht, welches in situ vollständig im Rektum aufgenommen wird, während der transanale Abschnitt nur aus einer einzigen Schlauchlage dieses Ballons besteht, wird bei der Ausführungsform nach Fig. 7 ein davon getrenntes, vorzugsweise versteifendes Element verwendet. Bei der Fig. 8 wird im transanalen Abschnitt eine Schlauchlage des Ballons einerseits mit einem davon getrennten Versteifungselement verwendet. Die Ausführungsform gemäß fig. 9 verfügt im transanalen Bereich über zwei zueinander konzentrische Schlauchlagen, welche ein Kompartiment begrenzen, welches von dem intrarektalen Ballonkompartiment vollständig getrennt ist und daher unabhängig von dem anderen Kompartiment befüllt werden kann, bspw. um eine Abdichtung zu erreichen. Bei der Ausführungform nach Fig. 10 ist innerhalb des transanalen Kompartiments, welches von dem intrarektalen Kompartiment vollständig getrennt ist, zusätzlich ein Versteifungselement enthalten. Demgegenüber ist bei der Kopfeinheit 201 der zwischen den zueinander konzentrischen Lagen des zu einem Ballon rückgestülpten Schlauchabschnitts verbleibende Zwischenraum nicht in zwei Kompartimente unterteilt, sondern der intrarektale Hohlraum kommuniziert mit dem transanalen Hohlraum. Andererseits können die beiden Schlauchlagen punkt-, linien- oder flächenartig miteinander verschweißt sein, um bspw. ein drainierendes Lumen offenzuhalten.

**Bezugszeichenliste**

| | | | |
|---|---|---|---|
| 1 | Spül-Kathetersystem | 17 | Konnektorelement |
| 2 | Retentionsballon | 17a | Zylinder |
| 2a | rektale Erweiterung | 17b | Zylinder |
| 2b | prä-anale Erweiterung | 18 | Filterelement |
| 2c | trans-anale Verjüngung | 19 | Gaze |
| 3 | zentrale Öffnung | 20 | Dichtungslippe |
| 4 | Schlauchfolienelement | 21 | Element |
| 4a | ableitender Schenkel | 22 | Hülle |
| 4b | zuleitender Schenkel | 23 | Umfangbereiche |
| 4c | Schaftschlauch | 101 | intrarektales Ballonsegment |
| 4d | Schaftkomponente | 102 | transanales Ballonsegment |
| 5 | Spül- und Sammelbeutel | 103 | Trichterelement |
| 5c | Kompartiment | 104 | Verbindungsschlauch |
| 5d | Kompartiment | 105 | Entleerungsschlauch |
| 6 | ventilartige Einheit | 106 | Kompartiment |
| 7 | Funktionselement | 107 | Präformation |
| 8 | Einlasselement | 123 | ringartiges Element |
| 9 | netzartige Trennfolie | 124 | ringnutartige Präformation |
| 10 | verschließbare Öffnung | 125 | Rohr- oder Schlauchstück |
| 11 | Rohrkörper | 126 | proximales Ende |
| 12 | Öffnungen | 127 | äußere Hülle,Folienschlauch |
| 13 | Schlauchelement | 128 | Kompartiment |
| 14 | IN-Skala | 129 | Taillierung |
| 15 | OUT-Skala | 130 | Rohrgeflecht |
| 16 | Halterung | 131 | Zuleitung |
| 201 | Vorrichtung | | |
| 202 | präformierter Schlauch | | |
| 203 | radiale Erweiterung | | |
| 204 | radiale Erweiterung | | |
| 205 | Schlauchabschnitt | | |
| 206 | Schlauchabschnitt | | |
| 207 | freies Ende | | |
| 208 | freies Ende | | |
| 209 | Konnektorelement | | |
| 210 | Hohlraum | | |
| 211 | zylindrischer Abschnitt | | |
| 212 | Schweißverbindung | | |
| 213 | inneres Lumen | | |
| 214 | Versteifungshülse | | |
| 215 | Schlauch | | |
| 216 | Okklusionsballon | | |
| 217 | Schlauch | | |
| 218 | Schlauch | | |
| 219 | Innenhülse | | |
| 220 | Kanal | | |
| S | Set-basierte Ausführung | | |
| SB | Spül- und Sammelbeutel | | |
| SG | Abschlusselement | | |
| SK | Kopfeinheit | | |
| SLB | Auffangbeutel | | |

## Patentansprüche

1. Vorrichtung zur Durchführung einer lavage-artigen, repetitiven Zu- und Ableitung einer spülenden, reinigenden oder sonstigen therapeutisch wirksamen Substanz, zu/von einem Patienten, insbesondere im Rahmen einer groß-lumig spülenden bzw. ableitenden Reinigung des Colons eines Patienten, umfassend:
- eine dauerhaft atraumatisch anorektal platzierbare, stuhlaufnehmende und -ableitende Kathetereinheit (SK);
- eine Spül- und Sammelbeuteleinheit (5,SB) mit kombinierter Spül-und Sammelfunktion; sowie
- eine die Kathetereinheit (SK) mit der Spül- und Sammelbeuteleinheit (5,SB) verbindende Schlaucheinheit (4), umfassend einen ein- oder mehrlumigen, folienartigen, flach kollabierbaren Schlauch;
**gekennzeichnet durch** wenigstens ein Element mit einer flussrichtungsabhängigen Drosselfunktion, welches dazu konfiguriert ist, dass die Ableitung von Darminhalt vom Patienten her derart großlumig erfolgt, dass auch geformte Stuhlanteile effizient abgeleitet werden, während die Zuleitung von Flüssigkeit zum Patienten hin im Verhältnis zu der großlumigen Ableitung von Darminhalt gedrosselt erfolgt und somit die Strömungsmenge oder -geschwindigkeit bei der Zuleitung auf ein organverträgliches, nicht-irritierendes Maß reduziert.

2. Vorrichtung nach Anspruch 1, **dadurch gekennzeichnet, dass** sowohl der Zustrom von der Spül- und Sammelbeuteleinheit (5,SB) zum Patienten hin, als auch der Abstrom vom Patienten zur Spül- und Sammelbeuteleinheit (5,SB) durch Ventile oder Einwegventile, oder durch ventilartige Funktions- oder Steuerelemente flussgerichtet sind.

3. Vorrichtung nach Anspruch 1, **dadurch gekennzeichnet, dass** lediglich der Abstrom vom Patienten zur Spül- und Sammelbeuteleinheit (5,SB) durch ein Ventil oder ein Einwegventil oder durch ein ventilartiges Funktions- oder Steuerelement flussgerichtet ist.

4. Vorrichtung nach einem der vorhergehenden Ansprüche, **dadurch gekennzeichnet, dass** die Zuleitung zum Patienten entweder über dasselbe Lumen erfolgt wie die Ableitung, oder durch ein getrenntes Lumen mit einem relativ zum ableitenden Lumen kleineren Querschnitt.

5. Vorrichtung nach einem der vorhergehenden Ansprüche, **gekennzeichnet durch** ein Element mit einer stuhlabscheidenden Funktion, das es erlaubt, im Beutel (5,SB) geformte Anteile aus bereits in den Beutel abgeleitetem Darminhalt zu separieren und dort gefangen zu halten und lediglich den Rückstrom von flüssigen Anteilen des repetitiv zu- und abgeleiteten Mediums aus dem Beutel zum Patienten gestattet.

6. Vorrichtung nach Anspruch 5, **dadurch gekennzeichnet, dass** das abscheidende Element eine netzartige, eine gewebeartige oder eine siebartig perforierte Struktur aufweist, welche Flüssigkeit oder in Flüssigkeit suspendiertem Stuhl passieren lässt, geformte Stuhlanteile jedoch am Rückstrom zum Patienten hindert.

7. Vorrichtung nach Anspruch 5 oder 6, **dadurch gekennzeichnet, dass** der Beutel (5,SB) eine Trennlage (TL) aufweist, die entweder zwei Kompartimente (5c, 5d) vollständig voneinander separiert, oder als stuhlabscheidende oder -filternde, netzartige, vliesartige oder siebartig perforierte Struktur ausgebildet ist, welche flüssige von festen Stuhlanteilen trennt.

8. Vorrichtung nach Anspruch 5 bis 7, **dadurch gekennzeichnet, dass** innerhalb des Beutels (5), in endständiger Verlängerung der verbindenden Schlaucheinheit (4) mit einlumigem, kombiniert zu- und ableitender Aufbau, ein Element (12) mit einer flussreduzierenden Funktion und ein Element (13) mit einer flussrichtenden Funktion integriert oder angeordnet sind.

9. Vorrichtung nach einem der vorhergehenden Ansprüche, **dadurch gekennzeichnet, dass** die den Analkanal über seine gesamte Länge hinweg durchsetzende und abdichtende Kathetereinheit (SK) als ein- oder doppelwandige Ballonkomponente (2) ausgebildet ist.

10. Vorrichtung nach Anspruch 9, **dadurch gekennzeichnet, dass** die den Analkanal über seine gesamte Länge hinweg durchsetzende und abdichtende Kathetereinheit (SK) aus durch Blasformung auf das anatomisch erforderliche Maß ausgeformtem Polyurethan besteht.

11. Vorrichtung nach einem der vorhergehenden Ansprüche, **dadurch gekennzeichnet, dass** die ein- oder doppelwandige Ballonkomponente (2) an ihrer Außenseite hantelförmig ausgebildet ist, derart, dass die taillenartige Verjüngung den Analkanal durchsetzt, während eine distale Erweiterung intrarektal angeordnet ist und eine proximale Erweiterung extrakorporal.

12. Vorrichtung nach einem der vorhergehenden Ansprüche, **dadurch gekennzeichnet, dass** innerhalb einer hantelförmigen Ballonkomponente eine die Ballonkomponente tragende, transanal platzierbare, stuhlableitende Schlauchkomponente (4d) angeordnet ist, welche insbesondere durch ein welliges Profil versteift ist und somit, in Relation zu einem nicht wellig ausgeformten Schlauch gleichen Durchmessers und Materials, vergleichbare elastische Aufrichtungseigenschaften bei geringeren Wandungsstärken ermöglicht.

13. Vorrichtung nach einem der vorhergehenden Ansprüche, **dadurch gekennzeichnet, dass** die verbindende Schlaucheinheit (4) und/oder ein an deren proximalem Ende angeordneter Konnektor (17) atraumatisch, bei leichter Krafteinwirkung von außen flach kollabierbar ist, um somit die Entstehung von Druckstellen bei einem eventuellen Aufliegen des Patienten zu verhindern.

14. Vorrichtung nach Anspruch 1, **dadurch gekennzeichnet, dass** die Schlaucheinheit (4) proximal in ein ebenfalls atraumatisch kollabierbares Konnektorelement (17) übergeht, woran die Spül- und Sammelbeuteleinheit (5,SB) lösbar angeschlossen oder anschließbar ist.

15. Vorrichtung nach Anspruch 14, **gekennzeichnet durch** eine an dem Konnektorelement (17) optional anschließbare, extrakorporale Sammel- und/oder Entgasungseinheit.

## Claims

1. Device for conducting a lavage-like, repetitive supply and drain of a flushing, cleaning or other therapeutically active substance, to/from a patient, especially within the scope of a large-lumen rinsing or, respectively, draining cleaning of the colon of a patient, comprising:
- a permanently atraumatic anorectally placeable catheter unit (SK), for receiving and draining off the feces;
- a rinsing and collecting bag unit (5,SB) with combined rinsing and collecting function; as well as
- a hose unit (4) connecting said catheter unit (SK) with said rinsing and collecting bag unit (5,SB), comprising a single or multi-lumen film-like flat collapsible hose;
**characterized by** at least one element with a throttle function dependent from the flow direction, which is configured for draining intestinal content from a patient by use of such a large lumen, that even formed feces are drained efficiently, while the supply of fluid directed to the patient is throttled relative to the large-lumen draining of intestinal content and thereby, at the supply, the flow rate or the flow velocity is reduced to a not irritating value which is compatible with the body.

2. Device according to claim 1, **characterized in that** the flow directions of the inflow from the rinsing and collecting bag unit (5,SB) directed to the patient as well as of the outflow directed from the patient towards the rinsing and collecting bag unit (5,SB) are adjusted through valves or one-way valves or through the valve-type functional or control elements.

3. Device according to claim 1, **characterized in that** only the direction of the flow directed from the patient to the rinsing and collecting bag unit (5,SB) is adjusted through a valve-type functional or control element.

4. Device according to one of the preceding claims, **characterized in that** the supply directed to the patient takes place either through the same lumen like the draining or through a separate lumen with a smaller cross section in relation to the draining lumen.

5. Device according to one of the preceding claims, **characterized by** an element with a feces-separating function which allows to separate formed feces from intestinal contents already drained into the bag and keeps them inside and allows only the backflow of fluid components of the repetitive supply and draining medium from the bag to the patient.

6. Device according to claim 5, **characterized in that** the separating element comprises a net-like, a fabric-like or a sieve-like perforated structure, which allows fluid or stool particles suspended in fluid to pass, but prevents any backflow of formed feces to the patient.

7. Device according to claim 5 or 6, **characterized in that** the bag (5,SB) comprises a separation layer (TL), which either separates two compartments (5c, 5d) from each other completely, or is formed as a feces retaining or filtering net-like, fleece-like or sieve-like perforated structure, which separates fluid and solid feces parts.

8. Device according to one of claims 5 to 7, **characterized in that** an element (12) with a flow reducing function and an element (13) with a flow directing function are integrated or arranged within the bag (5) as a terminal extension of the connecting hose unit (4) having a single-lumen construction combined for supply and draining.

9. Device according to one of the preceding claims, **characterized in that** the catheter unit (SK) penetrating and sealing the anal canal along its entire length is made as a single- or double-walled balloon component (2).

10. Device according to claim 9, **characterized in that** the catheter unit (SK) penetrating and sealing the anal canal along its entire length is made by blow molding from polyurethane shaped into the anatomically necessary dimension.

11. Device according to one of the preceding claims, **characterized in that** the single- or double-walled balloon component (2) is dumbbell-shaped at its outer side, in such a way, that the waist-like tapering penetrates the anal canal, while a distal extension is arranged intrarectally and a proximal extension is arranged extracorporeally.

12. Device according to one of the preceding claims, **characterized in that** a trans-anally placeable stool draining hose component (4d) supporting the balloon components is arranged within a dumbbell-shaped balloon component, which hose component (4d) is especially reinforced by a corrugated profile and thereby allows comparable elastic straightening properties at lower wall thicknesses in relation to a hose having the same diameter and being made from the same material without a corrugated profile.

13. Device according to one of the preceding claims, **characterized in that** the connecting hose unit (4) and/or a connector (17) attached to its proximal end is/are atraumatic collapsible upon application of a small external force, in order to prevent the development of pressure sores in case of a patient possible lying on it.

14. Device according to claim 1, **characterized in that** said connecting hose unit (4) proximally transforms in an atraumatic collapsible connector element (17), to which the rinsing and collecting bag unit (5, SB) is detachably connected or connectable.

15. Device according to claim 14, **characterized by** an extracorporeal collecting and/or degassing unit, which is optionally connectable to said connector element (17).

## Revendications

1. Dispositif pour la réalisation d'une administration et d'une évacuation répétitive, de type lavement, d'une substance de rinçage, de nettoyage ou présentant un autre effet thérapeutique, vers/depuis un patient, en particulier dans le cadre d'un nettoyage à effet de rinçage ou d'évacuation par l'intermédiaire d'une lumière de grand diamètre du côlon d'un patient, comprenant:
- une unité de cathéter (SK) pouvant être placée à demeure de manière atraumatique et anorectale recevant et évacuant les selles;
- une unité de poche de rinçage et de collecte (5, SB) avec une fonc- tion combinée de rinçage et de collecte; ainsi qu'
- une unité de flexible (4) reliant l'unité de cathéter (SK) à l'unité de poche de rinçage et de collecte (5, SB), comportant un flexible pliable à plat, de type film, simple ou double lumière; **caractérisé par** au moins un élément ayant une fonction d'étranglement en fonction du sens de flux, qui est conçu de manière à ce que l'évacuation du contenu de l'intestin du patient s'effectue par l'intermédiaire d'une lumière de grand diamètre de telle sorte que même des parties de selles formées sont évacuées efficacement, pendant que l'administration de liquide vers le patient par rapport à l'évacuation par l'intermédiaire d'une lumière de grand diamètre du contenu de l'intestin s'effectue de manière étranglée et réduit ainsi le débit ou vitesse d'écoulement lors de l'administration à un niveau compatible avec les organes et non irritant.

2. Dispositif selon la revendication 1, **caractérisé en ce que** tant le flux entrant de l'unité de poche de rinçage et de collecte (5, SB) vers le patient que le flux sortant du patient vers l'unité de poche de rinçage et de collecte (5, SB) sont dirigés dans le sens du flux par des vannes ou des vannes monovoie, ou par des éléments fonctionnels ou de commande de type vanne.

3. Dispositif selon la revendication 1, **caractérisé en ce que** seul le flux sortant du patient vers l'unité de poche de rinçage et de collecte (5, SB) est dirigé dans le sens du flux par une vanne ou une vanne monovoie ou par un élément fonctionnel ou de commande de type vanne.

4. Dispositif selon l'une des revendications précédentes, **caractérisé en ce que** l'administration vers le patient s'effectue soit par l'intermédiaire de la même lumière que l'évacuation, ou par l'intermédiaire d'une lumière séparée présentant une section plus petite par rapport à la lumière d'évacuation.

5. Dispositif selon l'une des revendications précédentes, **caractérisé par** un élément ayant une fonction de séparation des selles qui permet de séparer des parties formées dans la poche (5, SB) à partir du contenu de l'intestin déjà évacué dans la poche et de les y collecter et d'autoriser uniquement le reflux de parties liquides du fluide administré et évacué de manière répétitive de la poche vers le patient.

6. Dispositif selon la revendication 5, **caractérisé en ce que** l'élément de séparation présente une structure perforée de type filet, tissu ou tamis, qui laisse passer le liquide ou des selles en suspension dans le liquide, mais qui empêche toutefois le reflux de parties de selles formées vers le patient.

7. Dispositif selon la revendication 5 ou 6, **caractérisé en ce que** la poche (5, SB) comporte une couche de séparation (TL) qui sépare complètement l'un de l'autre soit deux compartiments (5c, 5d) ou qui est réalisée sous forme de structure perforée séparatrice ou filtrante de selles, ou de type filet, voile ou tamis, laquelle structure sépare des parties de selles liquides des parties de selles solides.

8. Dispositif selon la revendication 5 à 7, **caractérisé en ce qu'**un élément (12) ayant une fonction de réduction de flux et un élément (13) ayant une fonction de direction de flux sont intégrés ou disposés à l'intérieur de la poche (5), dans le prolongement terminal de l'unité de flexible (4) de liaison ayant une structure simple lumière combinant administration et évacuation.

9. Dispositif selon l'une des revendications précédentes, **caractérisé en ce que** l'unité de cathéter (SK) traversant et assurant l'étanchéité du canal anal sur toute sa longueur se présente sous forme de composant de ballonnet (2) à simple ou double paroi.

10. Dispositif selon la revendication 9, **caractérisé en ce que** l'unité de cathéter (SK) traversant et assurant l'étanchéité du canal anal sur toute sa longueur est constitué de polyuréthane moulé par soufflage à la dimension anatomique nécessaire.

11. Dispositif selon l'une des revendications précédentes, **caractérisé en ce que** le composant de ballonnet (2) à simple ou double paroi sur sa face extérieure est réalisé sous forme de haltère, de telle sorte que le rétrécissement de type taille traverse le canal anal, tandis qu'un élargissement distal est disposé de manière intrarectale et un élargissement proximal de manière extracorporelle.

12. Dispositif selon l'une des revendications précédentes, **caractérisé en ce qu'**un composant de flexible (4d) porteur, pouvant être placé de manière transanale, évacuant les selles du composant du ballonnet est disposé dans un composant de ballonnet en forme de haltère, ledit composant de flexible étant renforcé en particulier par un profilé ondulé et ainsi, par rapport à un flexible non ondulé de même diamètre et de même matériau, permettant des propriétés de redressement élastiques comparables à épaisseurs de paroi plus faibles.

13. Dispositif selon l'une des revendications précédentes, **caractérisé en ce que** l'unité de flexible (4) de liaison et/ou un connecteur (17) disposé à son extrémité proximale peut être de manière atraumatique plié à plat sous l'effet d'une légère force extérieure afin d'empêcher l'apparition de points de pression en cas d'un éventuel appui du patient.

14. Dispositif selon la revendication 1, **caractérisé en ce que** l'unité de flexible (4) se transforme de manière proximale en un élément de connecteur (17) pouvant également être plié de manière atraumatique, auquel l'unité de poche de rinçage et de collecte (5, SB) est raccordée ou peut être raccordée de manière désolidarisable.

15. Dispositif selon la revendication 14, **caractérisé par** une unité de collecte et/ou de dégazage extracorporale pouvant être raccordée en option à l'élément de connecteur (17).
